Europäisches Patentamt

European Patent Office

Office européen des brevets

(11) Publication number: **0 311 273**
**A1**

## (12) EUROPEAN PATENT APPLICATION

(21) Application number: 88308734.8

(51) Int. Cl.⁴: **C12N 15/00**

(22) Date of filing: **21.09.88**

The applicant has filed a statement in accordance with Rule 28 (4) EPC (issue of a sample only to an expert). Accession number(s) of the deposit(s): CMI 317734, CMI 314652, NCIB 12510.

The title of the invention has been amended (Guidelines for Examination in the EPO, A-III, 7.3).

(30) Priority: **24.09.87 GB 8722478**
**27.01.88 GB 8801766**

(43) Date of publication of application:
**12.04.89 Bulletin 89/15**

(84) Designated Contracting States:
**BE CH DE FR GB IT LI NL**

(71) Applicant: **BEECHAM GROUP PLC**
**Beecham House Great West Road**
**Brentford Middlesex TW8 9BD(GB)**

(72) Inventor: **Bull, John Henry University of Bristol**
**Department of Microbiology The Medical School**
**University Walk Bristol BS8 1TD(GB)**
Inventor: **Smith, David John University of Bristol**
**Department of Microbiology The Medical School**
**University Walk Bristol BS8 1TD(GB)**
Inventor: **Turner, Geoffrey University of Bristol**
**Department of Microbiology The Medical School**
**University Walk Bristol BS8 1TD(GB)**

(74) Representative: **West, Vivien et al**
**Beecham Pharmaceuticals Great Burgh Yew Tree Bottom Road**
**Epsom Surrey KT18 5XQ(GB)**

(54) **Transformation system for Penicillium.**

(57) A transformation system for transforming P. chrysogenum host organisms and selecting the transformants produced involves conferring oligomycin resistance on the host by integration of a suitable homologous DNA sequence, especially a gene forming part of the chromosomal DNA of a mutant strain of P. chrysogenum which has acquired oligomycin resistance.

Fig. 2

## NOVEL PROCESS

This invention relates to a transformation system for introducing exogenous DNA into Penicillium chrysogenum, to a process for preparing and selecting transformed Penicillium chrysogenum strains using the transformation system and to P. chrysogenum transformants prepared by the process.

Penicillium chrysogenum is a filamentous fungus of considerable commercial importance since it produces the important antibiotics penicillin G and penicillin V. The exploitation of recombinant DNA techniques using P. chrysogenum as a host has, however, been made extremely difficult by the lack of suitable cloning vectors and transformation methods. Without a suitable selection system, identification of transformants, which are produced only with low frequency in P. chrysogenum, is difficult if not impossible.

Transformation has been described for a number of filamentous fungi and has typically depended on the availability of auxotrophic mutants, for which the corresponding wild-type gene, in plasmid-borne form, provides a selectable marker. Transformation of P. chrysogenum via this approach has recently been reported (Sanchez et al., Gene, 1987 51, 97-102) using the wild-type trp C gene to relieve an auxotrophic tryptophan-requiring mutant. A problem with this method, however, is that transformants could only be selected from strains carrying the auxotrophic mutation.

Recently reports of filamentous fungal transformation using dominant selectable genes have appeared. The amdS gene of Aspergillus nidulans is capable not only of A. nidulans transformation but also of transforming other fungal species including P. chrysogenum (Beri and Turner, Current Genetics, 1987, 11, 639-641), and can, in principle, be selected on any ascomycete or related filamentous fungal species lacking the ability to grow on acetamide.

European Patent Application Publication Numbers 0 215 539 and 0 260 762 also disclose methods for preparing and selecting Penicillium transformants. The latter document (unpublished at the priority date of the present invention) mentions in general terms the possibility of using a gene providing resistance to oligomycin to select the transformed host; however all the examples relate to complementation of an auxotrophic host with structural genes coding for an enzyme in a metabolic pathway.

Oligomycin is a hydrophobic antibiotic which inhibits mitochondrial ATP synthase (Enns and Criddle, Arch. Biochem. Biophys., 1977, 182, 587-600).

Nuclear oligomycin - resistant mutants of Neurospora crassa have been shown to contain an ATP synthase having an altered subunit 9 amino acid sequence [Sebald et al, see "Mitochondria 1977- Genetics and Biogenesis of Mitochondria" Bandlow W. et al (eds), W. de Gruyter, Berlin]. The subunit 9 gene, in its altered oligomycin-resistant form, has been cloned from a mutant of Aspergillus nidulans (Ward et al., Mol. Gen. Genet. 1986, 202, 265-270) and direct selection of oligomycin resistance following transformation of wild-type A. nidulans has been achieved.

Hitherto the transformation of P. chrysogenum using a homologous oligomycin resistance gene as a selectable marker has not been described.

As used herein the term 'homologous' as applied to a gene or DNA sequence is used to denote a gene or DNA sequence found in P. chrysogenum.

According to the present invention there is provided a method for transforming Penicillium chrysogenum and for selecting the transformants produced, which method comprises the steps of :

i) mixing, under transforming conditions, a P. chrysogenum host organism and a recombinant vector comprising a homologous DNA sequence coding for oligomycin resistance; and

ii) selecting one or more organisms which grow when cultured in a medium comprising oligomycin.

It will be appreciated that the DNA sequence which codes for oligomycin resistance should be capable of being integrated into, and expressed in, P. chrysogenum.

From the foregoing it will be understood that in an alternative aspect the present invention provides a transformed strain of Penicillium which is selectable in an oligomycin-containing medium, characterised in that the strain is Penicillium chrysogenum, and in that the genome of the transformed strain has integrated therein a homologous DNA sequence coding for oligomycin resistance.

The advantage of the invention is that it permits effective transformation and convenient and rapid identification of oligomycin resistant transformants of P. chrysogenum strains.

The host cell may be any suitable strain of P. chrysogenum, for example P. chrysogenum NRRL 1951 (wild type) or a nicotinamide auxotroph of the wild type, for example CMI 317734 deposited at the Commonwealth Mycological Institute, Ferry Lane, Kew, Surrey, England on 22nd July 1987.

Recombinant vectors suitable for use in the invention may be derived by cloning a homologous DNA sequence which codes for oligomycin resistance into any suitable vector, for example plasmid or phage DNA.

Preferred vectors are plasmids, especially those comprising an E. coli origin of replication and a sequence which codes for a selectable phenotype in E. coli.

Particular plasmids of this type include pUC9 (Viera and Messing, Gene, 1982, 19, 259-268).

Further suitable vectors include cosmids, for example pHC 79 (Hohn and Collins, Gene, 1980, 11, 291-298).

In a preferred aspect, the homologous DNA sequence which codes for oligomycin resistance is part of the chromosomal (nuclear) DNA of a mutant strain of P. chrysogenum which has acquired oligomycin resistance. The homologous DNA sequence may suitably comprise any gene coding for a protein which interferes with the mechanism by which oligomycin exerts a fungistatic effect on the untransformed P. chrysogenum host.

Suitably the gene codes for an altered subunit of ATP-synthase of P. chrysogenum, especially for an altered subunit-9 protein.

Preferably the DNA sequence comprising the gene coding for an altered subunit-9 protein of the ATP-synthase of P. chrysogenum is obtained from an oligomycin-resistant mutant strain of P. chrysogenum produced by mutation of a nicotinamide auxotroph of the wild type strain (P. chrysogenum NRRL 1951).

A mutant oligomycin-resistant strain particularly suitable for the above purpose is that having the designated P. chrysogenum CMI 314652, which was deposited under the terms of the Budapest Treaty at the Commonwealth Mycological Institute on 16th April, 1987.

P. chrysogenum CMI 314652 forms another aspect of this invention.

The derivation and characteristics of P. chrysogenum CMI 314652 are as follows.

Derivation of P. chrysogenum CMI 314652

P. chrysogenum CMI 317734 underwent a spontaneous mutation to oligomycin resistance, giving organisms capable of vigorous growth on medium containing oligomycin at 5μg/ml. The mutant strain was indistinguishable from the parent strain on drug-free medium.

A heterokaryon test was carried out to check that the mutation was located in the nuclear genome (see Rowlands and Turner, Mol. Gen. Genet. 1973, 126, 201-216 for method).

Taxonomy of P. chrysogenum CMI 314652

A wild type strain of Penicillium chrysogenum - (designated NRRL 1951), isolated at the Northern Regional Research Laboratories, Peoria, Illinois, U.S.A. (Raper and Alexander, Journal of the Elisha Mitchell Scientific Society, 1945, 61, 74-113) was the parent of the strains used throughout this work.

In order to obtain the DNA sequence coding for oligomycin resistance from the chromosomal DNA of a mutant oligomycin resistant strain of P. chrysogenum it is first necessary to construct a 'library' of chromosomal DNA fragments which may then be 'probed' to isolate a suitable DNA fragment.

The library may be prepared by:

(a) partial digestion of chromosomal DNA with a suitable restriction endonuclease, for example Sau 3A;

(b) size fractionation to give fragments of appropriate length, suitably 5-40kb, preferably 10-20kb;

(c) ligation of the fragments into a vector; and

(d) transformation of a suitable host by the recombinant DNA produced.

Any suitable vector may be used in the construction of the library.

In a preferred aspect of the invention the lambda vector EMBL4 (Frischauf et al, J. Mol. Biol., 1983, 170, 827-842) is used as the vector and size-fractionated fragments of P. chrysogenum CMI 314652 chromosomal DNA are inserted into the Bam HI sites in order to construct the library of chromosomal DNA fragments.

Standard procedures may be used for all the above DNA manipulations [see Maniatis et al, "Molecular cloning, A Laboratory Manual" (1982), CSHL Press, New York].

Any convenient host may be used for constructing the library. Preferred hosts include E. coli, for example E. coli DH1 which may be transformed according to the method of Hanahan (J.Mol. Biol., 1983, 166, 557-580).

Clones in the library containing the DNA sequence coding for oligomycin resistance may be identified by standard hybridisation techniques (see Maniatis et al., loc cit.) using a suitable probe.

The probe may suitably be an oligodeoxynucleotide having at least 13 bases, the sequence of which is complementary, or partially complementary, to a portion of the known sequence of a gene coding for oligomycin resistance.

When the DNA sequence coding for oligomycin resistance comprises the gene coding for an altered subunit-9 protein of ATP-synthase the probe is suitably complementary to a portion of the known sequence of the oligomycin-resistance gene (oliC31) of A. nidulans (Ward and Turner, Mol. Gen. Genet., 1986, 205, 331-338). Alternatively the probe may comprise the whole oli C31 gene which may optionally be incorporated into a suitable vector.

A preferred probe is the plasmid pMW11 containing the A. nidulans pre-subunit 9 DNA (Ward et al., Mol. Gen. Genet., 1986, 202, 265-270).

It will be understood that the probe is labelled so that clones in the library containing complementary DNA may be readily identified.

Normally the probe is radio-labelled, for example with $^{32}P$. Radio-labelling may be carried out by standard techniques, for example 5'-or 3'-end labelling, including nick-translation.

Clones in the library designated as 'positive' (i.e. copies of which are found to hybridise to the probe) may be isolated and DNA coding for oligomycin restance may be obtained therefrom by excision with one or more suitable restriction endonucleases.

In order to define clearly further aspects of the present invention, reference is made to the accompanying drawings in which:

Figure 1(a) is a restriction map of DNA (I), a piece of P. chrysogenum CMI 314652 chromosomal DNA plus lamda EMBL4 vector DNA of total length 45kb;

Figure 1(b) is a restriction map of DNA (II), a 12.8kb Hind III fragment derived from DNA (I);

Figure 1(c) is a restriction map of DNA (III), a 9.3kb Hind III - Eco RI fragment derived from DNA (II);

Figure 1(d) is a restriction map of DNA (IV), a 3.8kb Hind III - Bam HI fragment derived from DNA (II) or DNA (III);

Figure 1(e) is a restriction map of plasmid pPOL1, constructed from DNA (II) and pUC9;

Figure 1(f) is a restriction map of plasmid pPOL6, constructed from DNA (III) and pUC9;

Figure 1(g) is a restriction map of plasmid pPOL8 constructed from DNA (IV) and pUC9;

Figure 2 is a Southern analysis of total DNA from 5 pPOL1 transformants (lanes 1-5) and P. chrysogenum CMI 317734 (lane 6) cleaved with Eco RI and probed with pUC9;

Figure 3(a) is a restriction map of the chromosomal region of the isopenicillin N synthetase gene (V) showing chromosomal Bam HI sites (vertical arrows) and the coding region (horizontal arrow);

Figure 3(b) is a restriction map of plasmid pCYX4, constructed from a 3.2 kb Xho I fragment of (V) by cloning it inter the Sal I site of pUC9;

Figure 3(c) is a restriction map of plasmid pPOI 1, constructed from an Eco RI fragment of the insert of pCYX4 subcloned into PPOL6; and

Figure 4 is a Southern analysis of pPOI 1 transformants I148 (track 1), I144 (track 2), I143 (track 3), and I142 (track 4) cut with Bam HI and I2421 cut with Xho I (track 5), Bam HI (track 6) and Eco RI (track 7); P. chrysogenum CMI 317734 DNA cut with Xho I (track 8), Bam HI (track 9) and Eco RI (track 10), probed with the pCYX4 Eco RI fragment encompassing the isopenicillin synthetase coding region of (V) shown in Figure 3(a).

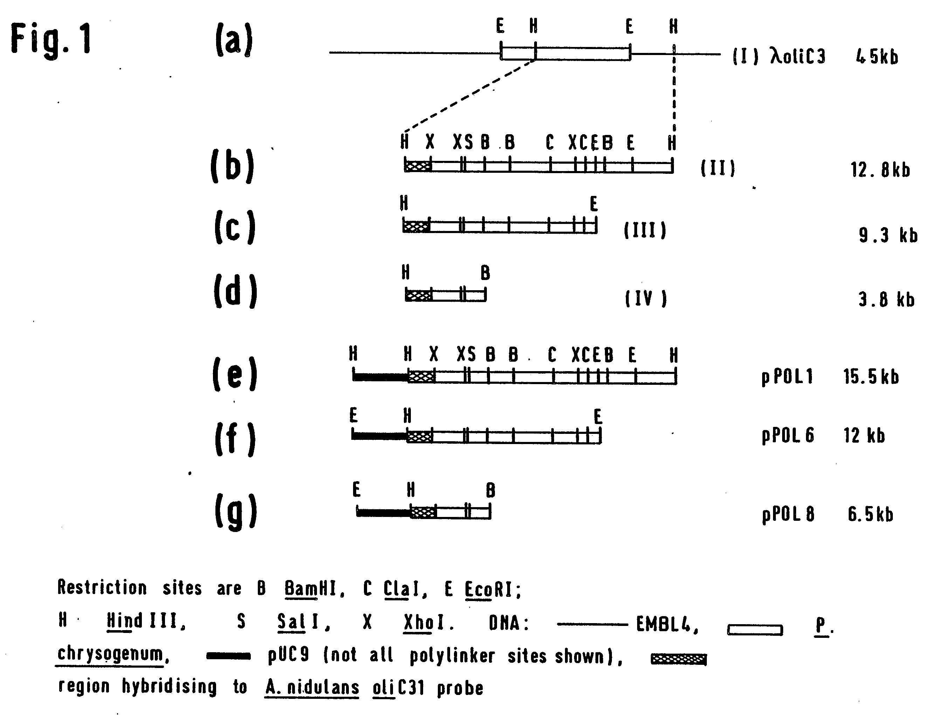

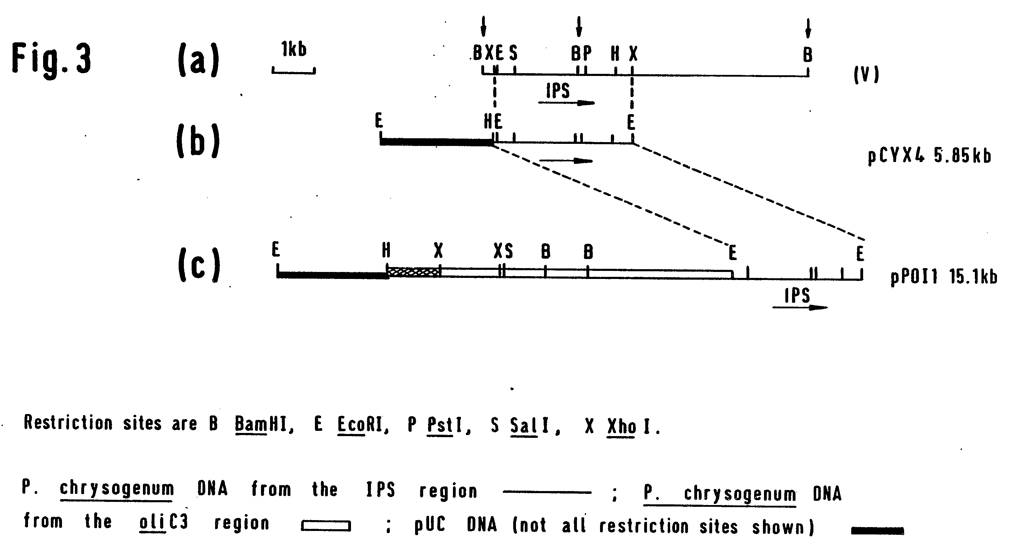

In Figures 1 and 3 not all restriction sites are necessarily shown.

From the foregoing, it will be appreciated that in a particularly preferred aspect of the invention the lambda EMBL4 library of P. chrysogenum CMI 314652 chromosomal DNA is probed with pMW11. A valuable piece of DNA which may be obtained by that procedure is designated oliC3 and has the configuration of restriction sites shown in Figure 1-(a).

Accordingly, according to another aspect of the invention, there is provided a piece of DNA (I), or a restriction fragment thereof containing an intact gene coding for oligomycin resistance, the said DNA having the configuration of restriction sites shown in Figure 1(a).

It will be understood that it is often inconvenient to use the whole length of DNA (I) shown in Figure 1(a) as the DNA coding for oligomycin resistance in the method according to the invention.

Accordingly, it is preferable to use a restriction fragment of the DNA (I) provided it contains an intact gene coding for oligomycin resistance.

Preferred restriction fragments include the 12.8kb Hind III fragment (II) having the configuration of restriction sites shown in Figure 1(b), the 9.3kb Hind III - Eco RI fragment shown in Figure 1-(c), and the 3.8kb Hind III - Bam HI fragment shown in Figure 1(d).

In a further aspect of the invention there is provided a recombinant vector which comprises the DNA (I), or a restriction fragment thereof containing an intact gene coding for oligomycin resistance.

Particular examples of the recombinant vector according to the invention are the following:

(a) pPOL1, a 15.5kb plasmid constructed from the DNA fragment (II) as hereinabove defined cloned into the Hind III site of pUC9 [see Figure 1-(e)];

(b) pPOL6, a 12kb plasmid constructed from DNA fragment (III) as hereinabove defined cloned into the Eco RI site of pUC9 [see Figure 1(f)]; and

(c) pPOL8, a 6.5kb plasmid constructed from the DNA fragment (IV) are hereinabove defined cloned into the Eco RI site of pUC9 [see Figure 1-(g)].

Under the terms of the Budapest Treaty E. coli (CS1 strain) transformed with pPOL6 was deposited at the National Collections of Industrial and Marine Bacteria, Aberdeen, Scotland on 22nd July, 1987, under the accession number NCIB 12510.

In addition to the homologous DNA sequence coding for oligomycin resistance, the recombinant vector suitable for use in the method of the invention will normally contain one or more additional cloned genes coding for one or more proteins which it is desired that the transformed P. chrysogenum host should produce. Such vectors form another aspect of the present invention.

In a further aspect of the present invention there is provided a· method for expressing an exogenous gene in P. chrysogenum which method comprises:

(a) mixing, under transforming conditions, a P. chrysogenum host with a recombinant vector comprising:

i) said gene, and

ii) a homologous DNA sequence which codes for oligomycin resistance;

(b) selecting oligomycin-resistant colonies; and

(c) allowing the said gene to be expressed therein.

The term 'exogenous gene' is used to denote any additional gene (homologous or heterologous) which is introduced into P. chrysogenum by the transformation methods herein described.

Additional cloned gene(s) may be introduced into recombinant vectors suitable for use in the method of the invention by standard cloning techniques. Such genes may be cloned into a suitable vector as hereinabove described before the DNA sequence which codes for oligomycin resistance is incorporated. Advantageously, however, the said one or more additional cloned genes are introduced into the recombinant vector comprising the DNA sequence which codes for oligomycin resistance.

A particular example· of an additional cloned gene which may be present in the recombinant vector is the isopenicillin N synthetase gene of P. chrysogenum. A particular recombinant vector incorporating the isopenicillin N synthetase gene is that designated as pPOI 1, which has the configuration of restriction sites shown in Figure 3(c).

The isopenicillin N synthetase gene may be isolated by using a synthetic oligonucleotide derived from the published sequence of the isopenicillin N synthetase gene of Cephalosporium acremonium (Samson et al., Nature, 1985, 318, 191-194 or P. chrysogenum (Carr et al., Gene, 1986, 48, 257-266) to probe a library of P.

chrysogenum DNA.

From the foregoing it will be appreciated that the invention also provides a transformed strain of Penicillium which is selectable in an oligomycin-containing medium, characterised in that the strain is Penicillium chrysogenum and in that the genome of the transformed strain has integrated therein

a) a homologous DNA sequence comprising a gene coding for oligomycin resistance; and

(b) an additional gene which can be expressed in P. chrysogenum.

Preferably the additional gene is the isopenicillin N synthetase gene of P. chrysogenum which may suitably be isolated and cloned in a vector as hereinabove described.

Transforming conditions suitable for use in the method of the invention include those known in the art. For example, transformation may suitably be effected in a medium comprising calcium chloride and polyethylene glycol.

Typically the strain of P. chrysogenum to be transformed will be transformed to oligomycin resistance at a frequency of 1-5 per $\mu$g of DNA ($10^{-5}$ to $10^{-6}$ transformants per viable protoplast).

In order to select transformed protoplasts, selection will normally be delayed until approximately 20 hours after plating, allowing regeneration of all viable protoplasts.

Selection may then be achieved by overlaying with medium comprising oligomycin, typically present in a concentration of about 3$\mu$g/ml. Incubation is normally continued at about 30°C for 5-8 days. Transformants may be selected and purified by streaking onto plates containing oligomycin.

The following examples illustrate the invention.

Examples

In the examples described below the materials and methods used were as follows:

(a) Strains

P. chrysogenum CMI 317734 an oligomycin-sensitive, nicotinamide requiring strain; P. chrysogenum CMI 314652: a nuclear oligomycin-resistant mutant derived from P. chrysogenum CMI 317734.

Escherichia coli strains. DH1 recA, palA, hsr, hsm+, endo1B, re1A1 was transformed according to Hanahan (J. Mol. Biol, 1983, 166, 557-580); MC1022 araD139, (ara leu) 7697, (lacZ)M15, ga1U, ga1K, StrA (Casadaban and Cohen, J. Mol. Biol. 1980, 138 179-207) was used in conjunction with pUC9 (Viera and Messing, Gene 1982, 19, 259-268).

## (b) Plasmids

The plasmid pMW11 was used for probing, which carries a fragment of DNA containing the oliC31 gene of Aspergillus nidulans (Ward et al, Mol. Gen. Genet. 1986, 202, 265-270).

## (c) Isolation and manipulation of DNA

Total P. chrysogenum DNA extraction from mycelium, and large scale plasmid preparations were as described by Ballance et al., Biochem. Biophys. Res. Comm., 1983, 112, 284-289. Standard procedures were used for DNA manipulations including small-scale plasmid isolation, fragment reisolation from agarose gels, ligation, Southern analysis and lambda library plaque hybridisation [Maniatis et al. "Molecular Cloning: A Laboratory Manual" (1982)].

For gene bank construction, total DNA from P. chrysogenum CMI 314652 was partially digested with Sau3A and size fractionated to give fragments of 10-20kb. These fragments were inserted into the BamHI cloning sites of the lambda vector EMBL4 (Frischauf et al., J. Mol. Biol., 1983, 170, 827-842).

## (d) Penicillium culture and manipulation

Standard Penicillium culture media were used, supplemented with appropriate nutrients according to the strain. Oligomycin (Sigma Chemical Co. Ltd., St. Louis, USA) was added as a 1000x stock in ethanol. Conidia collection, and Aspergillus media (complete -ACM, and minimal - AMM) were as described by Rowlands and Turner, Mol. Gen. Genet., 1972, 154, 311-317.

## (e) Penicillium transformation

Fresh Penicillium spore suspension (2ml) was added to 200ml AMM in a 1 litre conical flask and shaken at 25°C for about 16 hours. The resulting germinating conidia and young mycelia were collected by centrifugation (bench centrifuge) and washed in 0.9M NaCl, then resuspended in 15ml 0.9M NaCl containing 5mg/ml Novozym 234 (Novo industries, Bagsvaerd, Denmark) and gently shaken at 30°C for 1-1.5 hours when the extent of protoplasting was estimated by microcopic examination of osmotically lysed samples. Following formation of sufficient protoplasts (40-90%), they were filtered through nylon filter cloth (Gallenkamp, GMX-500-V) and sintered glass (porosity 1), washed twice in 0.9M NaCl, once in 1M Sorbitol, 50mM $CaCl_2$ and resuspended in 1M sorbitol, 50mM $CaCl_2$ at a concentration of 0.5 - 5x $10^8$/ml, and divided into 50ul aliquots. 1-5μg DNA (in up to 5μl 10mM Tris. HCl, 1mM EDTA, pH 7.5) was added, then 12.5μl of a freshly made, filtered-sterilised solution of 25% PEG 6000, 50mM $CaCl_2$, 10mM Tris.HCl, pH7.5 was added, mixing gently. Following incubation on ice for 20 minutes, 0.5ml of this PEG solution were added. Following incubation at room temperature for 5 minutes, 1ml 1M sorbitol, 50mM $CaCl_2$ was added, mixing by inversion. Aliquots were mixed with 10ml molten ACM containing 0.9M NaCl, and overlayed onto ACM plates. After incubation at 30°C for 20 hours, plates were overlayed with 25ml ACM containing 3μg/ml oligomycin. Incubation was continued at 30°C for a further 5-8 days, when transformed colonies begin to conidiate on the surface of the agar. Transformants were purified by streaking onto plates containing oligomycin.

## Example 1

### (a) Isolation of the gene and plasmid construction

The plasmid pMW11, containing the Aspergillus nidulans pre-subunit 9 DNA, was used as a probe against plaques of the lambda EMBL 4 library of total DNA from the oligomycin-resistant P.chrysogenum strain CMI 314652. Using a stringency allowing hydridisation of sequences with approximately 73% homology (55°C, 1xSSC), six plaques (from a total of about 20,000) hybridised strongly against a uniformly weak background, a frequency compatible with that expected assuming hybridisation to a single copy nuclear gene. One of these clones, λoli C3, was analysed by restriction mapping, and is shown in Fig. 1(a).

The 12kb Hind III fragment [Figure 1(b)], containing a segment of lambda DNA, was subcloned into pUC9 to form pPOL1 [Fig.1(e)]. The smaller plasmids pPOL6 and POL8 were made by subcloning a 9.3kb Hind III - Eco RI fragment [Figure 1(f)] and a 3.8kb Hind III - Bam HI fragment [Figure 1(g)] respectively.

### (b) Transformation of Penicillium

P. chrysogenum CMI 317734 was transformed to oligomycin resistance at a frequency of 1-5/μg DNA; $10^{-5}$ - $10^{-6}$ transformants per viable protoplast. All transformants tested showed increased resistance to oligomycin when subcultured onto selective medium.

The selection imposed on the transformed protoplasts was delayed until approximately 20h after plating, allowing regeneration of all viable protoplasts before overlaying with medium containing oligomycin. Transformants began to appear after 5-8 days.

Background growth of a small percentage of the regenerated protoplasts gradually occurs; plates where P. chrysogenum CMI 317734 was transformed with pUC9 showed some conidiating colonies after 8-10 days. These colonies fail to grow when subcultured onto oligomycin-containing medium, and similar colonies, appearing after 8-10 days, were observed when pPOL1 was used to effect transformation.

b) Phenotypes of transformants

Insertion of a single copy of the resistance gene into the genome would be expected to produce a semi-resistant transformant, since the wild-type sensitive allele is still present. Replacement of the wild-type gene, or insertion of many copies of the resistance gene would give a fully-resistant phenotype. A total of 3 out of 7 pPOL1 transformants tested showed similar growth rates to P. chrysogenum CMI 314652 when subcultured onto plates containing oligomycin. The remaining 4 showed a semi-resistant rate of growth. Fully-resistant sectors, analogous to those seen arising from Aspergillus semi-resistant transformants, where a gene-conversion event is thought to occur (Ward et al., Mol. Gen. Genet., 1986, 202, 265-270) were not seen. Plasmid pPOL6 gave 5/7 fully-resistant transformants.

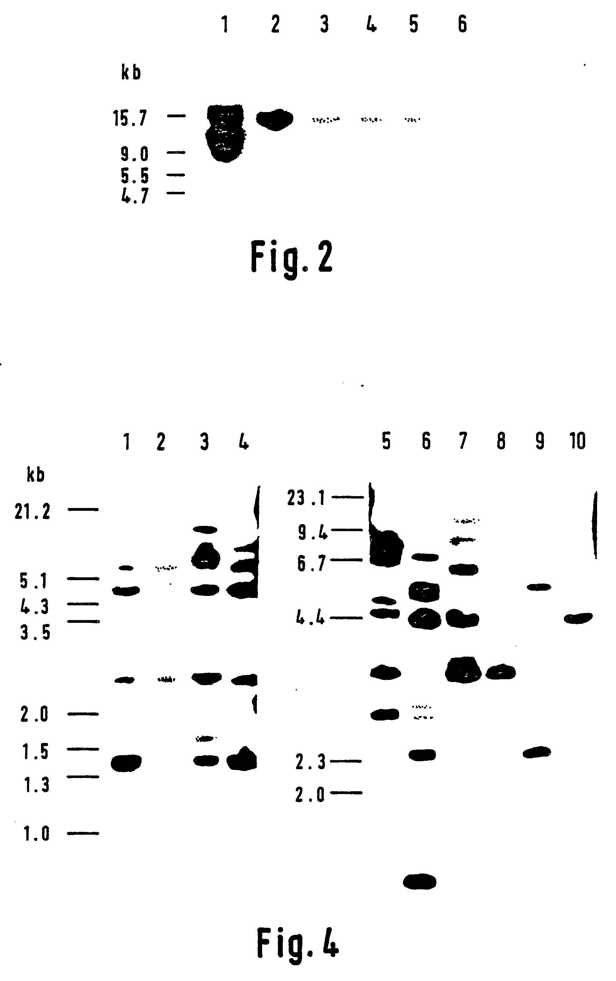

To demonstrate integration of pPOL1, several transformed lines were analysed for the presence of exogenous sequences by probing with pUC9 (Fig 2). Each transformant tested contains pUC sequences derived from the donor plasmid pPOL1.

Example 2

Insertion of the isopenicillin N synthetase gene from P. chrysogenum.

Plasmid pPOL6 is a derivative of pPOL1 which has a unique Eco RI site [Fig. 1(f)]. This site was used to insert a 3.2kb Eco RI fragment (containing a 3.2kb Eco RI-XhoI fragment) encompassing the P. chrysogenum isopenicillin N synthetase (IPS) gene, to form pPOI 1 [Fig.3(c)]. This gene was isolated by using a synthetic oligonucleotide, derived from the sequence of the Cephalosporium acremonium gene (Samson et al, Nature, 1985, 318, 191-194), to probe a gene bank of P. chrysogenum DNA. Authenticity of the cloned fragment was confirmed by sequence comparison to the P. chrysogenum IPS gene sequence published by Carr et al, Gene, 1986, 48, 257-266. DNA from four pPOI 1 transformants, cleaved with Bam H1, was analysed using the 3.2kb Eco R1 fragment containing the IPS coding region [Fig.3(b)] as a probe in Southern blots (Fig. 4). One of these, I144 (fully-resistant, track 2) gives only bands present in wild type (track 9), and has presumably been generated by homologous replacement of the sensitive allele by the donor DNA. Transformants I142, I143 and I148 (all semi-resistant) give extra hybridising bands due to further copies of the IPS sequence introduced by integration of pPOI 1 (tracks 1, 3 and 4). The pPO1 1 Bam H1 fragments expected to hybridise to this probe are of 1.3 and 6.0 kb [Fig 3-(c)], the former of which is present in all three transformants indicating the integrity of the Bam H1 site in the IPS coding region, while the latter may have been reduced to the 4.5kb band, again present in all three transformants. These two bands are stronger than the wild-type bands, indicating that multicopy integration may have occurred. In addition I142 and I143 contain weaker hybridising bands of unpredicted size. Xho 1, Bam H1 and Eco R1 digests of DNA from a further transformant I2421 (semi-resistant; tracks 5, 6 and 7) again gives those bands expected from the map of pPOI 1 [Xho 1 : 7.0kb, Eco R1 : 3.2kb, Fig 3(c)] in addition to the wild-type bands (see tracks 8, 9 and 10) and others.

These patterns are consistent with an integrated pPOI 1 sequence, often amplified, flanked by rearranged, single copy sequences.

## Claims

1. A transformed strain of Penicillium which is selectable in an oligomycin-containing medium, characterised in that the strain is Penicillium chrysogenum, and in that the genome of the transformed strain has integrated therein a homologous DNA sequence coding for oligomycin resistance.

2. A transformed strain as claimed in Claim 1 in which the homologous DNA sequence is part of the chromosomal DNA of a mutant strain of P. chrysogenum which has acquired oligomycin resistance.

3. A transformed strain as claimed in Claim 1 or Claim 2 in which the said DNA sequence codes for an altered subunit of ATP-synthase.

4. A transformed strain as claimed in Claim 3 in which the altered subunit of ATP-synthase is the subunit-9 protein.

5. A transformed strain as claimed in Claim 4 in which the DNA sequence is derived from an oligomycin-resistant strain of Penicillium chrysogenum produced by mutation of a nicotinamide-auxotroph of Penicillium chrysogenum NRRL 1951.

6. A transformed strain as claimed in Claim 5 in which the DNA sequence is derived from Penicillium chrysogenum CMI 314652.

7. A transformed strain as claimed in any one of Claims 1 to 6 in which the genome of the transformed strain additionally comprises an exogenous gene.

8. A transformed strain as claimed in Claim 7 in which the said gene is the isopenicillin N synthetase gene of Penicillium chrysogenum.

9. A recombinant vector comprising a DNA segment designated oliC3 or a restriction fragment thereof containing an intact gene coding for oligomycin-resistance.

10. A vector as claimed in Claim 9 selected from the group consisting of pPOL1, pPOL6 and pPOL8.

11. A vector as claimed in Claim 9 or Claim 10 comprising an additional cloned gene.

12. A vector as claimed in Claim 11 in which the additional cloned gene is the isopenicillin N-synthetase gene of Penicillium chrysogenum.

13. A strain of Penicillium chrysogenum transformed by a vector as claimed in any one of Claims 9 to 12.

14. A method for transforming Penicillium chrysogenum and for selecting the transformants produced, which method comprises the steps of:

i) mixing, under transforming conditions, a Penicillium chrysogenum host organism and a recombinant vector comprising a homologous DNA sequence coding for oligomycin resistance; and

ii) selecting one or more organisms which grow when cultured in a medium comprising oligomycin.

15. Penicillium chrysogenum CMI 314652.

# Fig. 1

**(a)**

(I) λoliC3   45kb

**(b)**

(II)   12.8kb

**(c)**

(III)   9.3 kb

**(d)**

(IV)   3.8 kb

**(e)**

pPOL1   15.5kb

**(f)**

pPOL6   12 kb

**(g)**

pPOL8   6.5kb

Restriction sites are B <u>Bam</u>HI, C <u>Cla</u>I, E <u>Eco</u>RI; H <u>Hind</u>III, S <u>Sal</u>I, X <u>Xho</u>I. DNA: ———— EMBL4, ▭ <u>P.</u> <u>chrysogenum</u>, ▬▬ pUC9 (not all polylinker sites shown), ▨ region hybridising to <u>A. nidulans</u> <u>oli</u>C31 probe

EP 0 311 273 A1

EP 0 311 273 A1

Fig. 2

Fig. 4

# Fig. 3

Restriction sites are B BamHI, E EcoRI, P PstI, S SalI, X XhoI.

P. chrysogenum DNA from the IPS region ——————— ; P. chrysogenum DNA from the oliC3 region ▭ ; pUC DNA (not all restriction sites shown) ▬▬▬

EP 0 311 273 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y P | EP-A-0 260 762 (GIST BROCADES) <br> * Claims 1,2; page 3, lines 10-11 * | 1-14 | C 12 N 15/00 |
| D,Y | MOLECULAR & GENERAL GENETICS, vol. 202, no. 2, 1986, pages 265-270, Springer-Verlag, Berlin, DE; M. WARD et al.: "Transformation of Aspergillus nidulans with a cloned, oligomycin-resistant ATP synthase subunit 9 gene" <br> * Pages 269-270 * | 1-14 | |
| Y | EP-A-0 225 128 (ELI LILLY) <br> * Claims 1,5,6,8,11,29; page 12, lines 21-38 * | 1-14 | |
| P,X | CURRENT GENETICS, vol. 13, no. 5, 1988, pages 377-382, Springer-Verlag, Berlin, DE; J.H. BULL et al.: "Transformation of Penicillium chrysogenum with a dominant selectable marker" <br> * Whole article * | 1-15 | TECHNICAL FIELDS SEARCHED (Int. Cl.4) <br><br> C 12 N |

The present search report has been drawn up for all claims

| Place of search | Date of completion of the search | Examiner |
|---|---|---|
| THE HAGUE | 13-01-1989 | WIESER, M.R.J. |